# EUROPÄISCHE PATENTSCHRIFT

(11) **EP 1 033 112 B1**
(45) Veröffentlichungstag und Bekanntmachung des Hinweises auf die Patenterteilung: **08.10.2003**
(21) Anmeldenummer: 99113073.3
(22) Anmeldetag: 06.07.1999
(51) Int. Cl.: A61B 17/72, A61F 2/02

(54) **Marknagel zur Knochendistraktion**
Intramedullary nail for bone distraction
Clou intramédullaire pour la distraction des os

(30) Priorität: 01.03.1999 DE 19908851
(43) Veröffentlichungstag der Anmeldung: 06.09.2000
(73) Patentinhaber: Baumgart, Rainer, Dipl.-Ing. Dr. med., D-81479 München (DE)
(72) Erfinder: Baumgart, Rainer, Dipl.-Ing. Dr. med., D-81479 München (DE)
(74) Vertreter: Finck, Dieter, Dr.Ing.

(56) Entgegenhaltungen:
- DE-A- 19 700 225
- FR-A- 2 267 080
- US-A- 5 626 579
- US-A- 5 792 076

## Beschreibung

Die Erfindung betrifft einen Marknagel zur Knochendistraktion mit einem in seinem Innenraum, vorzugsweise in seinem Innenraum im Bereich seines Eintreibendes, angeordneten elektromotorischen Antrieb, der über eine elektrische Verbindung mit einer Empfangsantenne zur Energieeinkoppelung verbunden ist.

Die Verwendung eines in den Knochenmarkraum einbringbaren Marknagels als aktives Implantat mit der Aufgabe einer gleichzeitigen Stabilisierung und Distraktion von operativ durchtrennten Röhrenknochen, insbesondere zum Zwecke der Verlängerung und zur Überbrückung von Knochendefekten, ist aus der DE 39 21 972 C2 und der DE 197 00 225 Basis für den Oberbegriff der Anspruchs 1 A1 bekannt.

Die DE 39 21 972 C2 erwähnt pauschal mechanische, pneumatische, hydraulische, elektrische, elektromagnetische und piezoelektrische Antriebe als im Inneren des Marknagels untergebrachte Kraftquelle.

Nach der DE 197 00 225 A1 wird ein aus der EP 0 320 621 bekanntes Planetenrollenspindelsystem als vorteilhaft angesehen.

Bei beiden Antriebssystemen wird die Energie von außen durch die Haut über eine Antenne eingekoppelt, die über ein Kabel mit dem Antrieb im Marknagelinneren in Verbindung steht.

Bekannt ist ferner eine Distraktionsvorrichtung, bei der nicht nur der Antrieb, sondern auch auch die gesamte Energiereserve und die Steuerung eingekapselt ist, so daß das System völlig autonom arbeitet und von außen nur noch zur Programmierung erreicht werden muß.

Die Kabelverbindung von Antenne und Antriebssystem stellt ein hochbelastetes Element dar. Am Femur erfolgt die Einbringung des Marknagels zwischen dem großen Rollhügel (Trochanter major) und dem Schenkelhals. Hier tritt auch das Kabel aus und führt zur Antenne, die meist subkutan zu liegen kommt. Bei jeder Beinbewegung vollzieht die Kabelaustrittsstelle eine Kreissegmentbewegung mit dem Drehzentrum im Hüftgelenk, womit das Kabel am Austritt aus dem Knochen einer Knickbelastung mit kleinstem Biegeradius ausgesetzt wird, zumal in diesem Bereich auch regelmäßig Verknöcherungen zu erwarten sind. Auch unter Verwendung hochflexibler Kabel, wie sie bei Herzschrittmachern üblich sind, ist eine Dauerfestigkeit nicht sicher gewährleistet. Problematisch ist auch die spätere Entfernung des Kabels, welches im Gewebe von einer feinen Bindegewebshülle eingescheidet wird. Bei Kabelbruch oder operativer Verletzung können unsterile Materialien in Verbindung mit der Körperflüssigkeit kommen.

An der Tibia ist die Distraktionsvorrichtung nach der DE 39 21 972 C2 (Fig. 1) nicht einsetzbar, da im proximalen Bereich anatomisch bedingt eine Krümmung des Marknagels (Herzogkrümmung) erforderlich ist, so daß der Marknagel durch die Distraktion nicht in den Knochen gezogen werden kann.

Geeignet wäre hier das Antriebssystem der DE 197 00 225 A1 (Fig. 2), weil der Antrieb erst distal der Krümmung zu liegen kommt. Jedoch ist auch hier die Führung des Kabels, das den Knochen unmittelbar vor dem Kreuzbandansatz verläßt und durch die Kniegelenkskapsel zur subkutan gelegenen Antenne verläuft, problematisch.

Würde man bei dem Marknagel nach der DE 39 21 972 C2 den Antrieb so ausführen, daß er distal zu liegen kommt, wäre zwar die Funktion gewährleistet, da der Marknagel nach proximal gleiten kann. Die Kabelverbindung nach außen müßte aber durch den Nagel oder außen am Nagel vorbei geführt werden, was technisch sehr aufwendig wäre.

Bei der eingangs genannten Ausführungsform, bei der nicht nur der Antrieb, sondern auch die gesamte Energiereserve und die Steuerung eingekapselt ist, ist das Problem der Kabelführung durch vollständige Integration aller Komponenten in das Marknagelinnere eliminiert. Jedoch sind die Energieressourcen begrenzt und das System ist störanfällig; ferner besteht die Möglichkeit, daß es sich verselbständigt.

Zusammenfassend ergibt sich, daß bei den bekannten Antriebssystemen wesentliche Nachteile sowohl bei einer im Körpergewebe frei verlaufenden Kabelverbindung zwischen den im Nagelinneren befindlichen Komponenten und der meist im Subcutangewebe gelegenen Antenne als auch bei der vollständigen Implantation aller Komponenten, also auch der Energieversorgung, in das Marknagelinnere bestehen.

Die der Erfindung zugrunde liegende Aufgabe besteht nun darin, die Marknägel der eingangs genannten Arten so auszugestalten, daß unter Beibehaltung der Distraktion unter Zug gemäß der DE 39 21 972 C2 oder unter Beibehaltung eines Teleskopmechanismus gemäß DE 197 00 225 A1 so zu gestalten, daß sowohl am Femur als auch an der Tibia eine Energieeinkoppelung gewährleistet ist, die sowohl die Nachteile einer vollständigen Integration der Energiereserven in das gekapselte System als auch einer Kabelverbindung zu einem subkutanen Empfängersystem vermeidet.

Diese Aufgabe wird ausgehend von den Marknägeln der eingangs genannten Arten dadurch gelöst, daß die Empfangsantenne und die elektrische Verbindung entweder gänzlich im Innenraum des Marknagels angeordnet sind und der Marknagel eine der Empfangsantenne zugewandte, die Energieeinkoppelung zulassende Öffnung aufweist, oder unmittelbar an der Stirnseite angebracht sind, so daß eine energetische Abschirmung durch das Metall des Marknagels nicht gegeben ist.

Bei dem erfindungsgemäß ausgestalteten Marknagel entfällt jede außerhalb des Marknagels vorzusehende Kabelverbindung. Gleichzeitig ist die erforderliche Energieeinkoppelung beispielsweise mit Hilfe eines Hochfrequenzsenders möglich, der bei entsprechend naher Positionierung die benötigte Energie problemlos übertragen kann. Dadurch stehen für den Antrieb immer die gewünschten Energieressourcen bereit, ohne daß sie innerhalb des Marknagels als solche installiert werden müssen. Die Öffnung kann mit einem die Energieeinkoppelung zulassenden Material wandbildend verschlossen werden, so daß der Marknagel eine durchgehend gleichbleibende Kontur hat.

Die Öffnung kann in der Wand des Marknagels ausgebildet sein. Wenn bei dieser Ausgestaltung der Antrieb, die Empfangsantenne und ihre elektrische Verbindung, gewöhnlich ein Kabel oder eine Steckverbindung, in einem gemeinsamen Gehäuse angeordnet sind, wird dieses in den Innenraum des Marknagels so eingesetzt, daß eine der Empfangsantenne zugewandte Öffnung in dem gemeinsamen Gehäuse der Öffnung in der Wand des Marknagels gegenüberliegt. Dadurch ist eine Vormontage von Antrieb, Empfangsantenne und elektrischer Verbindung in dem gemeinsamen Gehäuse möglich, ohne daß durch dieses gemeinsame Gehäuse eine die Energieeinkoppelung beeinträchtigende Abschirmung gegeben wäre. Die Öffnung in dem gemeinsamen Gehäuse kann natürlich auch von einem die Energieeinkoppelung zulassenden Material verschlossen werden, so daß das gemeinsame Gehäuse eine bündige Außenwand hat oder mit dem die Energieeinkoppelung zulassenden Material der Öffnung in der Wand des Marknagels durchgehend verbunden ist. Das gemeinsame Gehäuse kann aus Metall bestehen.

Als die Energieeinkoppelung zulassendes Material dient gewöhnlich ein körperverträgliches Expoxidharz oder Silikonkautschuk.

Zumindest die Empfangsantenne und meist auch ihre elektrische Verbindung können von einer gehäuseartigen Einkapselung aus die Energieeinkoppelung zulassendem Material umschlossen sein, wobei die Einkapselung in fester Verbindung mit dem elektromotorischen Antrieb stehen kann und mit oder ohne Antrieb in den Innenraum des Marknagels einführbar ist.

Um die fluchtende Ausrichtung der Empfangsantenne und der Öffnungen zu ermöglichen, können am Marknagel und an dem Gehäuse bzw. an der Einkapselung Justierelemente vorgesehen werden, beispielsweise in einer sich vom Eintreibende des Marknagels nach distal erstreckenden Aussparung und einer radial vom Gehäuse bzw. von der Einkapselung abstehenden Nase. In gleicher Weise können die Justierelemente aus der Öffnung in der Wand des Marknagels und einem von der Umfangsfläche der Einkapselung in Fluchtung zur Empfangsantenne abstehenden, in die Öffnung formschlüssig einschnappenden Ansatz bestehen, wenn dieser, wie die Einkapselung, von elastischem Silikonkautschuk gebildet wird.

Bei einer speziellen Ausgestaltung kann sich die Öffnung über die gesamte Umfangsfläche des Marknagels ausgehend von seinem Eintreibende in axialer Richtung nach distal erstrecken. In diesem Fall steht die Einkapselung aus dem die Energieeinkoppelung zulassenden Material axial über den Marknagel nach proximal vor und bildet eine die Empfangsantenne umschließende Kappe.

In allen genannten Fällen ist es möglich, stirnseitig an dem Marknagel ein geeignetes Werkzeug anzubringen, um den Marknagel im Knochen zu plazieren und ihn wieder zu entfernen.

Anhand von Zeichnungen werden Ausführungsbeispiele der Erfindung näher erläutert. Es zeigt:
- Fig. 1: perspektivisch das Eintreibende einer ersten Ausführungsform eines Marknagels,
- Fig. 2: perspektivisch wie Fig. 1 das Eintreibende einer zweiten Ausführungsform des Marknagels,
- Fig. 3: perspektivisch wie Fig. 1 das Eintreibende einer dritten Ausführungsform des Marknagels und
- Fig. 4: perspektivisch wie Fig. 1 das Eintreibende einer vierten Ausführungsform des Marknagels.
- Fig. 5: perspektivisch wie Fig. 1 das Eintreibende einer fünften Ausführungsform des Marknagels.

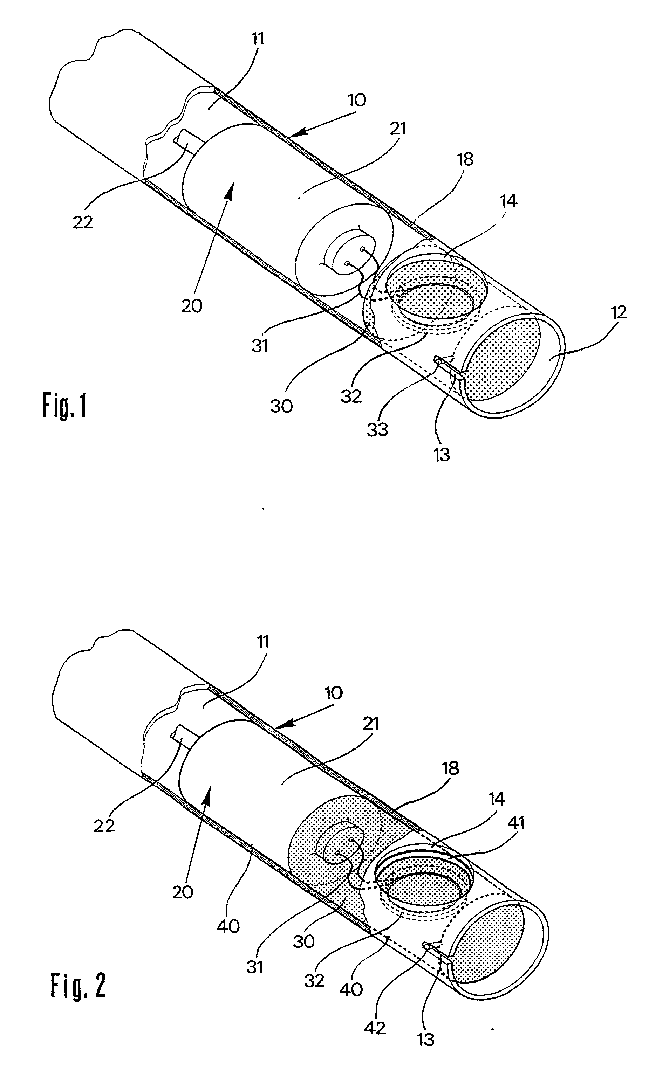

In den Figuren 1 bis 5 ist nur jeweils der Bereich am Eintreibende 12 eines Marknagels 10 mit einem Antrieb 20 gezeigt. Die Ausgestaltung des Distraktionsmechanismus des Marknagels 10 entspricht beispielsweise der in der DE 39 21 972 C2 oder DE 197 00 225 A1 beschriebenen Art.

Der aus Metall bestehende Marknagel 10 hat eine langgestreckte, im wesentlichen zylindrische Form mit einer Wand 18, die am Eintreibende 12 offen oder abgeschlossen ist. In dem hohlen Innenraum 11 des Marknagels 10 ist ein elektromotorischer Antrieb 20 vorgesehen, der in einem Antriebsgehäuse 21 eingekapselt und über eine Antriebswelle 22 mit dem nicht gezeigten Distraktionsmechanismus verbindbar ist. Der Antrieb 20 ist ferner über eine elektrische Verbindung, die als Kabel 31 dargestellt ist, mit einer in einer gehäuseartigen Einkapselung 30 untergebrachten Empfangsantenne 32 verbunden, die in dem eintriebsseitigen Endbereich des Marknagels 10 einer fensterartigen Öffnung 14 in der Wand 18 des Marknagels 10 gegenüberliegend angeordnet ist.

Um sicherzustellen, daß die Empfangsantenne 32 auch tatsächlich gegenüber der Öffnung 14 zu liegen kommt, ist in der Wand 18 an dem Eintreibende 12 des Marknagels 10 eine zur Stirnseite offene Aussparung 13 vorgesehen, in die eine Nase 33 der gehäuseartigen Einkapselung 30 der Empfangsantenne 32 formschlüssig eingreift.

Die für die jeweils vorzunehmende Verstellung des Distraktionsmechanismus des Marknagels 10 benötigte Energie wird von einem nicht gezeigten Sender aus über die im wesentlichen metallfreie Öffnung 14 zu der Empfangsantenne 32 übertragen, die die empfangene Energie mittels entsprechender elektronischer Bauteile in der erforderlichen Weise umwandelt und über das Kabel 31 in den elektromotorischen Antrieb 20 einspeist. Da sich die elektrische Verbindung in Form des Kabels 31 innerhalb des Marknagels 10 befindet, also kein Kabel außerhalb des Marknagels 10 vorgesehen werden muß, kann der Marknagel sowohl am Femur als auch an der Tibia problemlos eingesetzt werden.

In der Ausgestaltung von Fig. 2 sind der elektromotorische Antrieb 20, die elektrische Verbindung in Form des Kabels 31 und die Empfangsantenne 32 in einem gemeinsamen Gehäuse 40 aus Metall angeordnet, das mit einer fensterartigen Öffnung 41 versehen ist. Diese kapselartige Anordnung wird entweder mit der aus ihr herausragenden Antriebswelle 22 durch Einführen in den Innenraum 11 des Marknagels 10 mit dem Distraktionsmechanismus in Antriebsverbindung gebracht oder steht mit dem Distraktionsmechanismus bereits vor dem Einbringen in den Marknagel in fester Verbindung, wobei in der endgültigen Stellung die Öffnung 41 des gemeinsamen Gehäuses 40 zu der Öffnung 14 in der Wand 18 des Marknagels 10 ausgerichtet ist, so daß die Energieeinkoppelung für die Empfangsantenne 32 durch die Öffnungen 14 und 41 ohne Beeinträchtigung durch metallische Abschirmung erfolgen kann. Dabei kann die Öffnung 41 mit einem die Energieeinkoppelung zulassenden Material, beispielsweise einem körperverträglichen Epoxidharz oder Silikonkautschuk verschlossen sein. Um sicherzustellen, daß die Öffnung 41 im Gehäuse 40 mit der Öffnung 14 in der Wand 18 des Marknagels 10 in der endgültigen Position fluchtend zu liegen kommt, kann eine Aussparung 13 in der Wand 18 ausgehend von der proximalen Stirnfläche des Marknagels 10 eine Nase 42 des Gehäuses 40 aufnehmen.

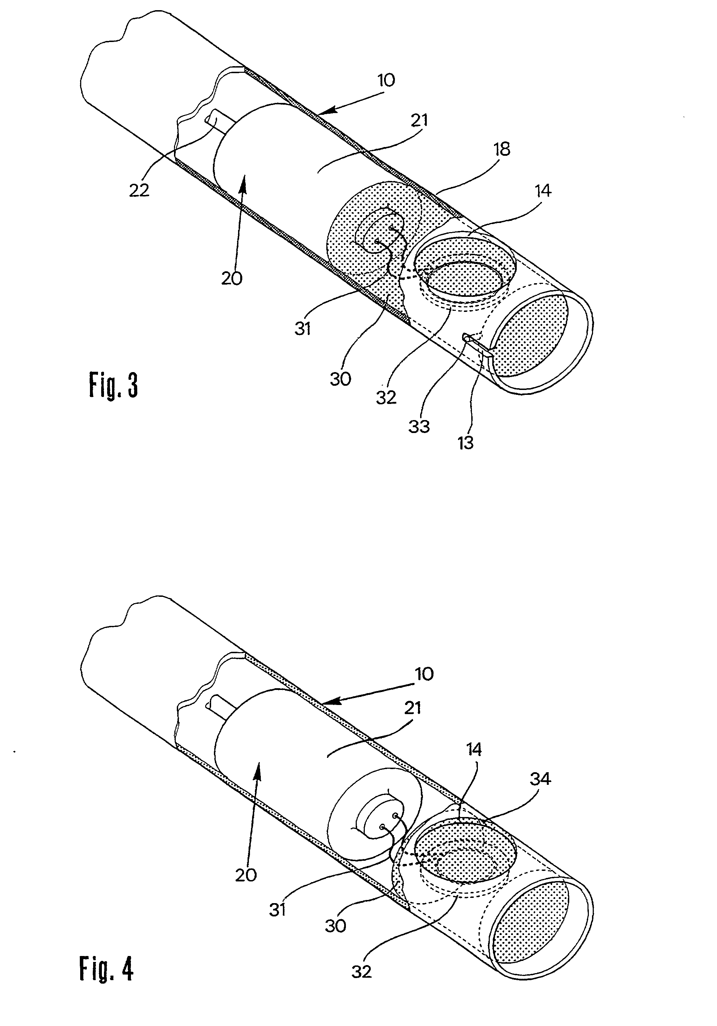

In der Ausgestaltung von Fig. 3 bilden der elektromotorische Antrieb 20, die elektrische Verbindung in Form eines Kabels 31 und die Empfangsantenne 32 eine Einheit, indem das Kabel 31 und die Empfangsantenne 32 in Epoxidharz oder Silikonkautschuk eingekapselt sind, wobei die so gebildete gehäuseartige Einkapselung 30 in fester Verbindung mit dem elektromotorischen Antrieb 20 steht. Auch hier wird durch eine Aussparung 13 im Marknagel 10 und durch eine Nase 33 an der Einkapselung 30 sichergestellt, daß die Empfangsantenne 32 und die Öffnung 14 des Marknagels 10 fluchten.

In der Ausgestaltung von Fig. 4 besteht die Einkapselung 30 für das Kabel 31 und die Empfangsantenne 32 aus flexiblem Silikonkautschuk und hat einen von ihrer Umfangsfläche als radiale Verlängerung der eingebetteten Empfangsantenne 32 abstehenden, in seiner Form der Öffnung 14 entsprechenden Ansatz 34, der formschlüssig in die Öffnung 14 des Marknagels 10 "einschnappen" kann. Dadurch ist sichergestellt, daß die Empfangsantenne 32 mit der Gehäuseöffnung 14 fluchtet.

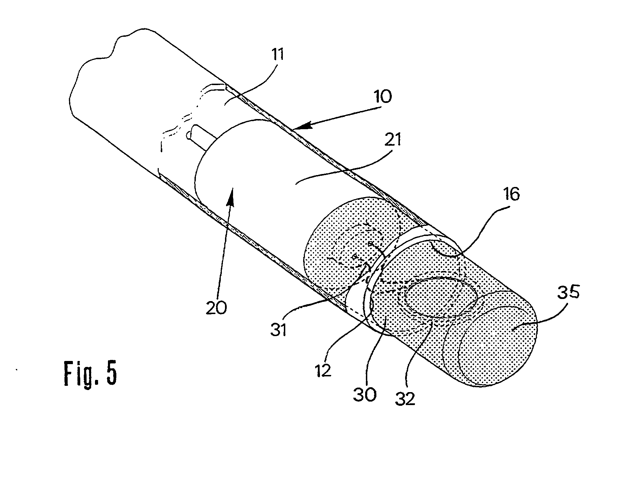

Die Ausführungsform von Fig. 5 benutzt als Öffnung für die Energieeinkoppelung die stirnseitige Öffnung 16 des Marknagels 10. Der elektromotorische Antrieb 20 ist dabei mit einer gehäuseartigen Einkapselung 30 fest verbunden, die vom Eintreibende 12 des Marknagels 10 her in den Innenraum 11 des Marknagels 10 eingeführt wird und in die die elektrische Verbindung in Form des Kabels 31 von der Empfangsantenne 32 zum elektromotorischen Antrieb 21 sowie die Empfangsantenne 32 eingebettet sind. Die Einkapselung 30 aus Epoxidharz oder Silikonkautschuk steht in Form einer Kappe 35, in der die Empfangsantenne 32 eingeschlossen ist, über die stirnseitige Öffnung 16 vor, so daß sich in diesem Fall die Öffnung 14 als sich über die gesamte Umfangsfläche des Marknagels 10 erstreckend interpretieren läßt.

### Bezugszeichenliste

- 10: Marknagel
- 11: Innenraum
- 12: Eintreibende
- 13: Aussparung
- 14: Öffnung
- 16: Öffnung (stirnseitig)
- 18: Wand

- 20: Antrieb
- 21: Antriebsgehäuse
- 22: Antriebswelle

- 30: Einkapselung
- 31: Kabel
- 32: Empfangsantenne
- 33: Nase
- 34: Ansatz
- 35: Kappe

- 40: Gehäuse (Metall)
- 41: Öffnung
- 42: Nase

## Patentansprüche

1. Marknagel (10) zur Knochendistraktion mit einem in seinem Innenraum (11), vorzugsweise in seinem Innenraum (11) im Bereich seines Eintreibendes (12), angeordneten elektromotorischen Antrieb (20), der über eine elektrische Verbindung (31) mit einer Empfangsantenne (32) zur Energieeinkoppelung verbunden ist, **dadurch gekennzeichnet, daß** die Empfangsantenne (32) und die elektrische Verbindung (31) gänzlich im Innenraum (11) des Marknagels (10) angeordnet sind und der Marknagel (10) eine der Empfangsantenne (32) zugewandte, die Energieeinkoppelung zulassende Öffnung (14) aufweist.

2. Marknagel (10) nach Anspruch 1, **dadurch gekennzeichnet, daß** die Öffnung (14) mit einem die Energieeinkoppelung zulassenden Material wandbildend verschlossen ist.

3. Marknagel (10) nach Anspruch 1 oder 2, **dadurch gekennzeichnet, daß** die Öffnung (14) in seiner Wand (18) ausgebildet ist.

4. Marknagel (10) nach Anspruch 3, **dadurch gekennzeichnet, daß** die Empfangsantenne (32) und ihre elektrische Verbindung (31) in einem gemeinsamen Gehäuse (40) angeordnet sind, das in den Innenraum (11) des Marknagels (10) so eingesetzt ist, daß eine der Empfangsantenne (32) zugewandte Öffnung (41) in dem gemeinsamen Gehäuse (40) der Öffnung (14) in der Wand (18) des Marknagels (10) gegenüberliegt.

5. Marknagel (10) nach Anspruch 4, **dadurch gekennzeichnet, daß** das Gehäuse (40) aus Metall besteht.

6. Marknagel (10) nach Anspruch 4 oder 5, **dadurch gekennzeichnet daß** die Öffnung (41) in dem gemeinsamen Gehäuse (40) von einem die Energieeinkoppelung zulassenden Material verschlossen ist.

7. Marknagel (10) nach einem der Ansprüche 1 bis 3, **dadurch gekennzeichnet, daß** wenigstens die Empfangsantenne (32) von einer gehäuseartigen Einkapselung (30) aus die Energieeinkoppelung zulassendem Material umschlossen ist, wobei die Einkapselung (30) für ein formschlüssiges Einführen in den Innenraum (11) des Marknagels (10) ausgebildet ist.

8. Marknagel (10) nach Anspruch 7, **dadurch gekennzeichnet, daß** die Empfangsantenne (32) und ihre elektrische Verbindung (31) von einer gehäuseartigen Einkapselung (30) aus die Energieeinkoppelung zulassendem Material umschlossen sind, die in fester Verbindung mit dem elektromotorischen Antrieb (20) steht und mit diesem in den Innenraum (11) des Marknagels (10) einführbar ist.

9. Marknagel (10) nach einem der vorhergehenden Ansprüche, **gekennzeichnet durch** die fluchtende Ausrichtung der Empfangsantenne (32) und der Öffnungen (14, 41) ermöglichende Justierelemente (13, 14, 42, 33, 34) am Marknagel (10) und an dem Gehäuse (40) bzw. an der Einkapselung (30).

10. Marknagel (10) nach Anspruch 9, **dadurch gekennzeichnet, daß** die Justierelemente von einer sich vom Eintreibende (12) des Marknagels (10) nach distal erstreckenden Aussparung (13) und einer radial vom Gehäuse (40) bzw. von der Einkapselung (30) abstehenden Nase (42 bzw. 33) gebildet werden.

11. Marknagel (10) nach Anspruch 9, **dadurch gekennzeichnet, daß** die Justierelemente von der Öffnung (14) und einem von der Umfangsfläche der Einkapselung (30) in Fluchtung zur Empfangsantenne (32) abstehenden, in die Öffnung (14) formschlüssig einschnappenden Ansatz (34) aus Silikonkautschuk gebildet werden.

12. Marknagel (10) nach Anspruch 8, **dadurch gekennzeichnet, daß** die Einkapselung (30) axial über den Marknagel (10) als eine die Empfangsantenne (32) umschließende Kappe (35) proximal vorsteht, so daß die Öffnung (14) sich über die gesamte Umfangsfläche des Marknagels (10) ausgehend von seinem Eintreibende (12) in axialer Richtung nach distal erstreckt.

13. Marknagel (10) nach einem der vorhergehenden Ansprüche, **dadurch gekennzeichnet, daß** das die Energieeinkoppelung zulassende Material Expoxidharz oder Silikonkautschuk ist.

## Claims

1. Intramedullary nail (10) for bone distraction, having arranged in its inner space (11), preferably in its inner space (11) in the region of its driving-in end (12), a drive (20), which is connected via an electrical connection (31) with a receiving antenna (32) for energy input, **characterised in that** the receiving antenna (32) and the electrical connection (31) are arranged entirely in the inner space (11) of the intramedullary nail (10) and the intramedullary nail (10) comprises an opening (14) facing towards the receiving antenna (32) and allowing the energy input.

2. Intramedullary nail (10) according to claim 1, **characterised in that** the opening (14) is closed, in wall-forming manner, with a material allowing the energy input.

3. Intramedullary nail (10) according to claim 1 or 2, **characterised in that** the opening (14) is constructed in its wall (18).

4. Intramedullary nail (10) according to claim 3, **characterised in that** the receiving antenna (32) and its electrical connection (31) are arranged in a common housing (40), which is inserted in the inner space (11) of the intramedullary nail (10) so that an opening (41) in the common housing (40) facing towards the receiving antenna (32) lies opposite the opening (14) in the wall (18) of the intramedullary nail (10).

5. Intramedullary nail (10) according to claim 4, **characterised in that** the housing (40) consists of metal.

6. Intramedullary nail (10) according to claim 4 or 5, **characterised in that** the opening (41) in the common housing (40) is closed by a material allowing the energy input.

7. Intramedullary nail (10) according to any one of claims 1 to 3, **characterised in that** at least the receiving antenna (32) is enclosed by a housing-like encapsulation (30) of material allowing the energy input, the encapsulation (30) being constructed for a interlocking insertion into the inner space (11) of the intramedullary nail (10).

8. Intramedullary nail (10) according to claim 7, **characterised in that** the receiving antenna (32) and its electrical connection (31) are enclosed by a housing-like encapsulation (30) of material allowing the energy input, which is in fixed connection with the electric motor drive (20) and is insertable together therewith into the inner space (11) of the intramedullary nail (10).

9. intramedullary nail (10) according to any one of the preceding claims, **characterised by** adjusting elements (13, 14, 42, 33, 34) on the intramedullary nail (10) and on the housing (40) and on the encapsulation (30) respectively, which adjusting elements permit the aligned orientation of the receiving antenna (32) and the openings (14, 41).

10. Intramedullary nail (10) according to claim 9, **characterised in that** the adjusting elements are formed by a cut-out (13) extending distally from the driving-in end (12) of the intramedullary nail (10) and by a projection (42, 33 respectively) projecting radially from the housing (40) and the encapsulation (30) respectively.

11. Intramedullary nail (10) according to claim 9, **characterised in that** the adjustment elements are formed by the opening (14) and an extension (34) of silicone rubber projecting in alignment with the receiving antenna (32) from the circumferential surface of the encapsulation (30) and engaging with an interlocking snap-fit in the opening (14).

12. Intramedullary nail (10) according to claim 8, **characterised in that** the encapsulation (30) projects proximally axially beyond the intramedullary nail (10) as a cap (35) enclosing the receiving antenna (32), so that the opening (14) extends distally in the axial direction over the entire circumferential surface of the intramedullary nail (10) starting from the driving-in end (12) thereof.

13. Intramedullary nail (10) according to any one of the preceding claims, **characterised in that** the material allowing energy input is epoxy resin or silicone rubber.

## Revendications

1. Clou intramédullaire (10) pour la distraction osseuse comprenant un entraînement commandé par moteur électrique (20) disposé dans son espace interne (11), de préférence dans son espace interne (11) dans la zone de son extrémité d'enfoncement (12), entraînement qui est relié, via une liaison électrique (31), à une antenne de réception (32) à des fins d'alimentation en énergie, **caractérisé en ce que** l'antenne de réception (32) et la liaison électrique (31) sont disposées complètement dans l'espace inteme (11) du clou intramédullaire (10) et le clou intramédullaire (10) présente une ouverture (14) tournée vers l'antenne de réception (32), permettant l'alimentation en énergie.

2. Clou intramédullaire (10) selon la revendication 1, **caractérisé en ce que** l'ouverture (14) est fermée en formant une paroi avec une matière permettant l'alimentation en énergie.

3. Clou intramédullaire (10) selon la revendication 1 ou 2, **caractérisé en ce que** l'ouverture (14) est pratiquée dans sa paroi (18).

4. Clou intramédullaire (10) selon la revendication 3, **caractérisé en ce que** l'antenne de réception (32) et sa liaison électrique (31) sont disposées dans un logement commun (40) qui est inséré dans l'espace interne (11) du clou intramédullaire (10) de telle sorte qu'une ouverture (41) tournée vers l'antenne de réception (32) dans le logement commun (40) est opposée à l'ouverture (14) pratiquée dans la paroi (18) du clou intramédullaire (10).

5. Clou intramédullaire (10) selon la revendication 4, **caractérisé en ce que** le logement (40) est constitué de métal.

6. Clou intramédullaire (10) selon la revendication 4 ou 5, **caractérisé en ce que** l'ouverture (41) dans le logement commun (40) est fermée par une matière permettant l'alimentation en énergie.

7. Clou intramédullaire (10) selon l'une quelconque des revendications 1 à 3, **caractérisé en ce qu'**au moins l'antenne de réception (32) est entourée par un encapsulage (30) en forme de logement constitué par la matière permettant l'alimentation en énergie, l'encapsulage (30) étant réalisé pour une insertion mécanique dans l'espace interne (11) du clou intramédullaire (10).

8. Clou intramédullaire (10) selon la revendication 7, **caractérisé en ce que** l'antenne de réception (32) et sa liaison électrique (31) sont entourées par un encapsulage (30) en forme de logement constitué par la matière permettant l'alimentation en énergie, encapsulage qui est relié à demeure à l'entraînement commandé par moteur électrique (20) et qui peut être introduit avec ce dernier dans l'espace interne (11) du clou intramédullaire (10).

9. Clou intramédullaire (10) selon l'une quelconque des revendications précédentes, **caractérisé par** des éléments d'ajustage (13, 14, 42, 33, 34) sur le clou intramédullaire (10) et sur le logement (40), resp. sur l'encapsulage (30), permettant l'alignement à fleur de l'antenne de réception (32) et des ouvertures (14, 41).

10. Clou intramédullaire (10) selon la revendication 9, **caractérisé en ce que** les éléments d'ajustage sont formés par un évidement (13) s'étendant vers l'extrémité distale à partir de l'extrémité d'enfoncement (12) du clou intramédullaire (10) et par un nez (42, resp. 43) en retrait en direction radiale par rapport au logement (40), resp. par rapport à l'encapsulage (30).

11. Clou intramédullaire (10) selon la revendication 9, **caractérisé en ce que** les éléments d'ajustage sont formés par l'ouverture (14) et par un prolongement (34) en caoutchouc de silicone en retrait par rapport à la surface périphérique de l'encapsulage (30) pour une mise à fleur avec l'antenne de réception (32) et venant s'encliqueter de manière mécanique dans l'ouverture (14).

12. Clou intramédullaire (10) selon la revendication 8, **caractérisé en ce que** l'encapsulage (30) fait saillie vers l'extrémité proximale en direction axiale au-delà du clou intramédullaire (10), sous la forme d'une coiffe (35) entourant l'antenne de réception (32), de telle sorte que l'ouverture (14) s'étend vers l'extrémité distale, sur toute la surface périphérique du clou intramédullaire (10), à partir de son extrémité d'enfoncement (12).

13. Clou intramédullaire (10) selon l'une quelconque des revendications précédentes, **caractérisé en ce que** la matière permettant l'alimentation en énergie est une résine époxy ou du caoutchouc de silicone.
